Europäisches Patentamt

European Patent Office

Office européen des brevets

(11) Publication number: **0 213 463 B1**

## EUROPEAN PATENT SPECIFICATION

(45) Date of publication of patent specification: **02.01.92**    (51) Int. Cl.5: **C12N 15/12**, C12P 21/02

(21) Application number: **86111056.7**

(22) Date of filing: **08.08.86**

(54) Interleukin 2 receptor and a method for its production.

(30) Priority: **13.08.85 JP 178429/85**

(43) Date of publication of application:
**11.03.87 Bulletin 87/11**

(45) Publication of the grant of the patent:
**02.01.92 Bulletin 92/01**

(84) Designated Contracting States:
**CH DE FR LI**

(56) References cited:
**EP-A- 0 162 699**

**NATURE, vol. 311, no. 5987, 18th October 1984, pages 631-635; T. NIKAIDO et al.: "Molecular cloning of cDNA encoding human interleukin-2 receptor"**

**PROC. NATL. ACAD. SCI. USA, vol. 80, November 1983, pages 6957-6961; W.J. LEONARD et al.: "Characterization of the human receptor for T-cell growth factor"**

**CHEMICAL ABSTRACTS, vol. 103, no. 25, 23rd December 1985, page 132, abstract no. 206667b, Columbus, Ohio, US; T.A. WALDMANN et al.: "Structure, function, and expression of the receptor for interleukin-2 on**

**normal and malignant lymphocytes", & CANCER CELLS 1985, 3(Growth Factors Transform.) 221-6**

(73) Proprietor: **Honjo, Tasuku
13-12, Ueno Higashi 3-chome
Toyonaka-shi Osaka(JP)**

(72) Inventor: **Honjo, Tasuku
No. 3-13-12, Uenohigashi
Toyonaka-shi Osaka-fu(JP)**
Inventor: **Shimizu, Akira Dept. of genetics
Harvard Med. School 25, Shttuck St.
Boston, MA 02115(US)**

(74) Representative: **Strehl, Schübel-Hopf, Groening
Maximilianstrasse 54 Postfach 22 14 55
W-8000 München 22(DE)**

**Description**

[Field of Industrial Application]

The present invention relates to soluble interleukin 2 receptor (hereafter referred to as soluble IL-2R, or sIL-2R which is released from the cortex cell and a method for the production thereof. The soluble IL-2R of the present invention is strongly intended to be used as an agent for the treatment of diseases induced by excessive immunity etc.

[Prior Art]

Interleukin 2 receptor (IL-2R) has been found as Tac antigen which is present on the cortex of T cells transformed by human adult leukemia T cell line (ATL) or human adult T cell leukemia virus (J. Exp. Med., 158, 1332-1337 (1983); Nature, 311, 631-635 (1984)) and takes a role for receiving IL-2 in the cortex or T cells and thus proliferating T cells.

The present inventors previously incorporated the gene coding for IL-2R into COS cell expression vector pKCRH 2. This plasmid was used to transform COS-7 cells, whereby Tac antigen, namely IL-2R, was detected on the cortex of the COS cells (Nature, 311, No. 5987, 631-635 (1984)). The entire base sequence of IL-2R DNA inserted into pKCRH 2 plasmid is determined and the coding is presumed.

[Problem Solved by the Invention]

IL-2R possesses the action to scavenge IL-2 but could not be administered to the patient because IL-2R has been hitherto produced in the form bound to the cell cortex. Accordingly, an object of the present invention is to provide free IL-2R released from the cell cortex, but with its biological activity retained, and a method for the production thereof.

[Means for Solving the Problem]

As a result of extensive investigations to solve the foregoing problems, the present inventors have succeeded in producing interleukin 2 receptor released from the cell cortex (sIL-2R) having the following properties:
(a) a molecular weight of 40 to 43 kD;
(b) an isoelectric point (pI) of 4.4 to 5.2 and
(c) the following amino acid sequence

X- Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

```
Trp-Glu-Asn-Glu-Ala-Thr-Glu-Arg-Ile-Tyr-His-

Phe-Val-Val-Gly-Gln-Met-Val-Tyr-Tyr-Gln-Cys-

Val-Gln-Gly-Tyr-Arg-Ala-Leu-His-Arg-Gly-Pro-

Ala-Glu-Ser-Val-Cys-Lys-Met-Thr-His-Gly-Lys-

Thr-Arg-Trp-Thr-Gln-Pro-Gln-Leu-Ile-Cys-Thr-

Gly-Glu-Met-Glu-Thr-Ser-Gln-Phe-Pro-Gly-Glu-

Glu-Lys-Pro-Gln-Ala-Ser-Pro-Glu-Gly-Arg-Pro-

Glu-Ser-Glu-Thr-Ser-Cys-Leu-Val-Thr-Thr-Thr-

Asp-Phe-Gln-Ile-Gln-Thr-Glu-Met-Ala-Ala-Thr-

Met-Glu-Thr
```

wherein X represents Met or a signal peptide having Met at the N-terminus.

The interleukin 2 receptor in secreted form may be produced according to this invention by culturing L cells or microorganisms belonging to the genus Escherichia tranformed with an expression vector carrying the DNA sequence coding for the interleukin 2 receptor in secreted form with the amino acid sequence

```
X-Glu-Leu-Cys-Asp-Asp-Asp-Pro-Pro-Glu-Ile-
  GAG CTC TGT GAC GAT GAC CCG CCA GAG ATC

Pro-His-Ala-Thr-Phe-Lys-Ala-Met-Ala-Tyr-Lys-
  CCA CAC GCC ACA TTC AAA GCC ATG GCC TAC AAG

Glu-Gly-Thr-Met-Leu-Asn-Cys-Glu-Cys-Lys-Arg-
  GAA GGA ACC ATG TTG AAC TGT GAA TGC AAG AGA

Gly-Phe-Arg-Arg-Ile-Lys-Ser-Gly-Ser-Leu-Tyr-
  GGT TTC CGC AGA ATA AAA AGC GGG TCA CTC TAT
```

```
Met-Leu-Cys-Thr-Gly-Asn-Ser-Ser-His-Ser-Ser-
ATG CTC TGT ACA GGA AAC TCT AGC CAC TCG TCC

Trp-Asp-Asn-Gln-Cys-Gln-Cys-Thr-Ser-Ser-Ala-
TGG GAC AAC CAA TGT CAA TGC ACA AGC TCT GCC

Thr-Arg-Asn-Thr-Thr-Lys-Gln-Val-Thr-Pro-Gln-
ACT CGG AAC ACA ACG AAA CAA GTG ACA CCT CAA

Pro-Glu-Glu-Gln-Lys-Glu-Arg-Lys-Thr-Thr-Glu-
CCT GAA GAA CAG AAA GAA AGG AAA ACC ACA GAA

Met-Gln-Ser-Pro-Met-Gln-Pro-Val-Asp-Gln-Ala-
ATG CAA AGT CCA ATG CAG CCA GTG GAC CAA GCG

Ser-Leu-Pro-Gly-His-Cys-Arg-Glu-Pro-Pro-Pro-
AGC CTT CCA GGT CAC TGC AGG GAA CCT CCA CCA

Trp-Glu-Asn-Glu-Ala-Thr-Glu-Arg-Ile-Tyr-His-
TGG GAA AAT GAA GCC ACA GAG AGA ATT TAT CAT


Phe-Val-Val-Gly-Gln-Met-Val-Tyr-Tyr-Gln-Cys-
TTC GTG GTG GGG CAG ATG GTT TAT TAT CAG TGC

Val-Gln-Gly-Tyr-Arg-Ala-Leu-His-Arg-Gly-Pro-
GTC CAG GGA TAC AGG GCT CTA CAC AGA GGT CCT

Ala-Glu-Ser-Val-Cys-Lys-Met-Thr-His-Gly-Lys-
GCT GAG AGC GTC TGC AAA ATG ACC CAC GGG AAG

Thr-Arg-Trp-Thr-Gln-Pro-Gln-Leu-Ile-Cys-Thr-
ACA AGG TGG ACC CAG CCC CAG CTC ATA TGC ACA

Gly-Glu-Met-Glu-Thr-Ser-Gln-Phe-Pro-Gly-Glu-
GGT GAA ATG GAG ACC AGT CAG TTT CCA GGT GAA

Glu-Lys-Pro-Gln-Ala-Ser-Pro-Glu-Gly-Arg-Pro-
GAG AAG CCT CAG GCA AGC CCC GAA GGC CGT CCT

Glu-Ser-Glu-Thr-Ser-Cys-Leu-Val-Thr-Thr-Thr-
GAG AGT GAG ACT TCC TGC CTC GTC ACA ACA CAA

Asp-Phe-Gln-Ile-Gln-Thr-Glu-Met-Ala-Ala-Thr-
GAT TTT CAA ATA CAG ACA GAA ATG GCT GCA ACC

Met-Glu-Thr
ATG GAG ACG
```

downstream of a promotor sequence, the expression vector having the capacity to proliferate in the host cell. The triplet coding for amino acid threonine 211 is immediately followed by a termination codon.

Additionally, the present invention provides a pharmaceutical composition, comprising a soluble interleukin 2 receptor protein (sIL-2R).

In case of L cells, the sIL-2R gene is arranged downstream of a Sin 40 early gene promoter of a vector capable of expressing in these cells, e.g., plasmid pKCRH 2 (Nature, 307, 604-608 (1984)). Using L cells it is preferred to clone the gene containing the sequence for the signed peptide with the following amino acid sequence:

```
Met – Asp-Ser-Tyr-Leu-Leu-Met-Trp-Gly-Leu-Leu-Thr-
      GAT TCA TAC CTG CTG ATG TGG GGA CTG CTC ACG

      Phe-Ile-Met-Val-Pro-Gly-Cys-Gln-Ala
      TTC ATC ATG GTG CCT GGC TGC CAG GCA
```

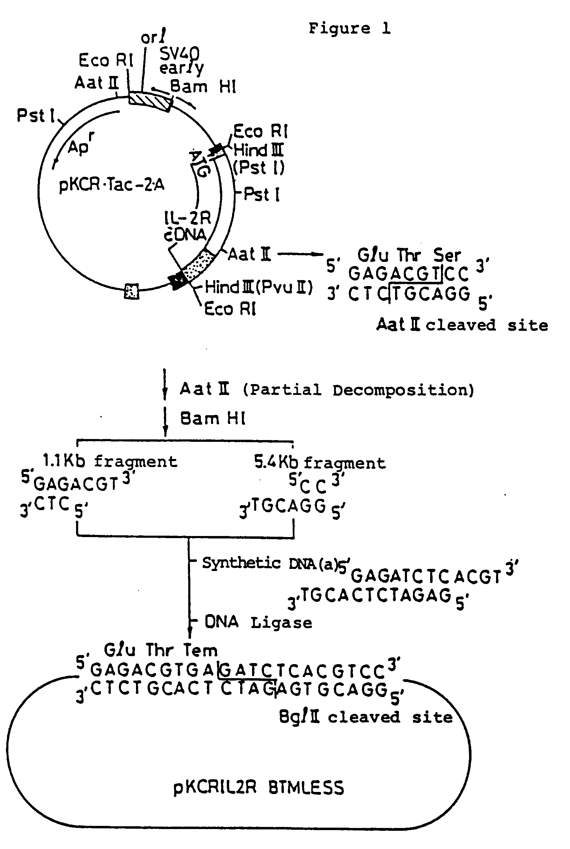

As may be recognized from figure 1, truncation of the receptor protein is mediated by insertion of a stop codon adjacent to the triplet encoding amino acid 211 (threonine, see Nature, 311, 631-635, (1984)) thus giving rise to sIL-2R lacking the 40 C-terminal amino acid residues as compared to physiological IL-2R. Concretely, the termination codon is introduced by inserting synthetic DNA(a) carrying 5'-phosphates shown in figure 1 into an Aat II restriction site of IL-2R cDNA into plasmid pKCR.Tac-2.A (Nature, 311, 631-635 (1984)). Thus, pKCRIL2R BTMLESS is constructed (cf. Figure 1) comprising the SV 40 early gene promoter, initiation codon ATG, a gene coding for a signal peptide and a gene coding for sIL-2R. In the case of the microorganism belonging to the genus Escherichia, the sIL-2R gene is placed into an expression vector of the microorganism with a promotor such as trp promoter, lac promoter, $p_L$ promoter of phage λ, etc., and a SD sequence downstream of it.

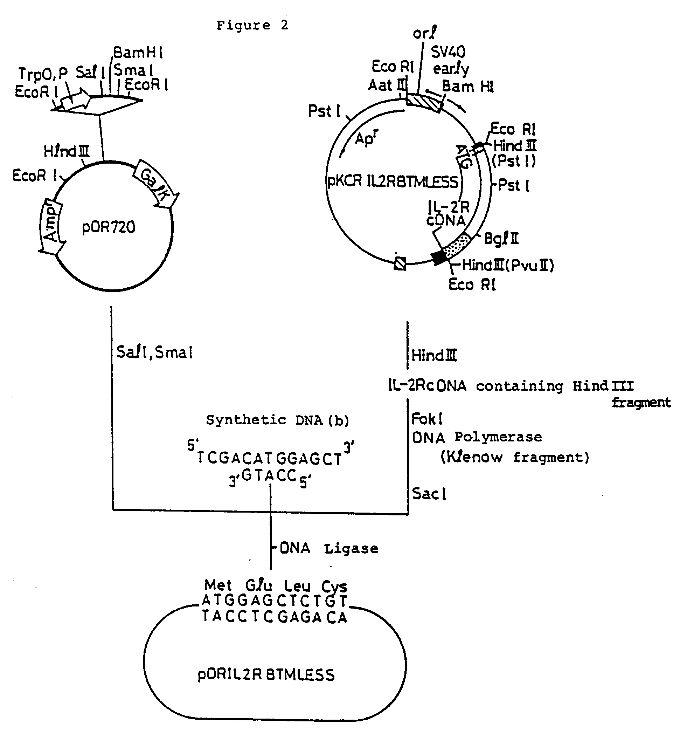

A specific example using trp promoter in Escherichia coli is shown below. pDR720 plasmid is cleaved by restriction enzymes Sal I and Sma I to give the linearized plasmid. The above-described pKCRIL2R BTMLESS plasmid is cleaved by restriction enzyme Hind III to give a fragment containing IL-2R cDNA. After further cleaving the fragment with restriction enzyme Fok I, it is treated with DNA polymerase (Klenow fragment) and cleaved by restriction enzyme Sac I. From the reaction products, the largest fragment is recovered. The thus recovered fragment, the 5'-terminal-phosphated synthetic DNA (b) (cf. Figure 2) and the pDR720 plasmid cleaved by restriction enzymes Sal I and Sma I are ligated with DNA ligase (Figure 2). Thus, pDRIL2R BTMLESS having arranged a SD sequence, an initiation codon ATG and the sIL-2R gene on the downstream of trp promoter can be constructed.

To transform a host cell using vector DNA bearing the sIL-2R gene, there are conventional methods for transformation shown below. In case that the host cells are L cells, there is a method for infecting DNA as calcium phosphate precipitates, a micro injection method, a method for inserting plasmid enclosed in red blood cells or ribosomes, a method for treating cells with reagents such as lysophosphatidylcholine, a method using virus vector, etc.

In case that the host cell is a microorganism belonging to the genus Escherichia, competent cells capable of taking up foreign DNA can be transformed, after recovering cells in the exponential growth phase, by the well known calcium chloride method. The efficiency of transformation can be improved in the co-presence of $MgCl_2$ or RbCl in the reaction solution after transformation. It is also possible to perform transformation after preparation of protoplast of the host cell.

Production of sIL-2R protein from transformed mouse L cells by incorporating the sIL-2R gene therein can be performed in accordance with a conventional method for culturing adherent cells. Namely, cultivation can be effected using a tissue culture flask such as Falcon 3024 or 3028 Flask (Falcon Labware, Div. Becton, Dickinson and Co.), while bubbling 5% $CO_2$. A roller flask such as Falcon 3027 can also be used. As medium, there may be used synthetic medium ordinarily used in tissue culture. Examples include Dulbecco's modified Eagle medium (DMEM), RPMI 1640 medium, etc. In the case of actually using these media, it is desired to supplement with 10% bovine fetal serum albumin, 100 units/ml of penicillin, 100 $\mu$g/ml of streptomycin and 2 g/l of $NaHCO_3$. The amount of sIL-2R protein produced by culturing the mouse L cells varies with passage of time. To produce the sIL-2R protein effectively, the following method is adopted. That is, culture is initiated in a cell density of $1 \times 10^5$/ml using DMEM medium (which contains 100 U/ml of penicillin, 100 $\mu$g/ml of streptomycin and 2 g/l of $NaHCO_3$) containing 10% FCS. When cells are dense after 4 to 5 days the supernatant is recovered and fresh medium is supplemented followed by culturing for 3 to 4 days. After completion of the culture, the medium is recovered for quantitative assay of the amount of sIL-2R protein produced. It becomes apparent that almost the same amount of the sIL-2R protein is produced in the former and latter cultivation.

sIL-2R can be purified from the thus obtained supernatant of the mouse L cells by affinity chromatography using IL-2-immobilized column, e.g., IL-2-Sepharose 4B or IL-2-Affi-Gel. To separate and purify in higher purity, reversed phase HPLC (high speed liquid chromatography) can be performed using an ODS column (octa decyl silane). The sIL-2R protein has the following properties:

(a) molecular weight: 43 - 40 kd (SDS-PAGE)

(b) pI: 5.2 to 4.4

(c) N-terminal amino acid sequence:

Glu-Leu-Cys-Asp-Asp-Asp-Pro-Pro-Glu-Ile-

The primary structure of the N-terminal amino acid was quite identical with the amino acid structure presumed from the base sequence of the IL-2R gene.

Example 1

As shown in Figure 1,

(1) 6 $\mu$g of plasmid pKCR.Tac-2.A (Nature, 311, 631-635 (1984)) having incorporated IL-2R cDNA in vector pKCRH2 containing a promoter for SV 40 early gene was partially decomposed with restriction enzyme Aat II and, straight chain plasmid fragments of about 6.5 kilobase (kb) were separated and recovered by agarose gel electrophoresis. The recovered fragments were 1 $\mu$g. The fragments were cleaved with restriction enzyme BamH I and, 0.35 $\mu$g of the 5.4 kb fragment and 0.07 $\mu$g of a 1.1 kb fragment were separated and recovered by agarose electrophoresis. An oligonucleotide having the sequence $^{5'}$GAGATCTCACGT$^{3'}$ was synthesized by a DNA automatic synthesis machine (made by Applied Bio System Co., Ltd., Model 380A). After purifying by reverse phase HPLC, the 5'-site was phosphated with $T_4$ polynucleotide kinase. In 10 $\mu$l of a reaction solution, 0.04 picomoles each of the 5.4 kb fragment and the 1.1 kb fragment and 0.16 picomoles of the aforesaid oligonucleotide (a) were maintained at 15°C for 1 hour. Then, the mixture was diluted to 100 $\mu$l. Ligation was further performed at 15°C for 12 hours using $T_4$ DNA ligase.

(2) Using 10 $\mu$l of this reaction solution, Escherichia coli HB101 cells were transformed in a conventional manner to give about 250 strains resistant to ampicillin. Twelve strains were optionally chosen therefrom and their plasmid DNAs were extracted. Then 5 transformants were obtained using plasmids satisfying the following 3 points that (1) the size of plasmid is about 6.5 kb, (2) the number of sites cleaved by restriction enzyme Aat II is one, and (3) the site cleavable by restriction enzyme Bge II derived from oligonucleotides (a) is present at the original site cleaved with restriction enzyme Aat II. The base sequence of the plasmid in the transformants was examined by the dideoxynucleotide chain termination method (Proc. Natl. Acad. Sci. U.S.A., 74, 5463 (1977)). It was confirmed that the plasmid had a base sequence of pKCRIL2R BTMLESS shown in Figure 1.

(3) From the thus obtained transformants, pKCRIL2R BTMLESS plasmid was purified by the cesium chloride equilibration centrifuge method. The purified plasmid was infected to mouse L cells (tk$^-$ mutant) (hereafter referred to as L cells) by the following method. That is, a suspension of 1 x 10$^5$ of L cells in 10 ml of 10% bovine fetal serum-containing Dulbecco's Eagle medium (DMEM) was charged in a Falcon 3003 dish followed by culturing at 37°C for 20 hours in a 5% carbon dioxide gas incubator. Then, the medium was replaced by 10 ml of fresh one followed by culturing for 4 hours under the same conditions. After the incubation, 0.5 ml of Solution A (50 mM Hepes-280 mM NaCl-1.5 mM sodium phosphate buffer, pH 7.22) and 0.5 ml of Solution B (2M $CaCl_2$-10 $\mu$g pKCRIL2R BTMLESS-1 $\mu$g pBR322-herpes Tk) were supplemented to the system followed by culturing at 37°C for 12 hours in a 5% carbon dioxide gas incubator. The culture was washed with TBS (0.137M NaCl-0.05M KCl-5.6 mM $Na_2HPO_4$-250 mm Tris-hydrochloride buffer (pH 7.5)) and then treated with a TBS solution containing 2.5% glycerol for 3 minutes. Immediately after the treatment, the system was washed with TBS, and 10 ml of DMEM containing 10% bovine fetal serum was added thereto followed by culturing at 37°C for 2 days in a 5% carbon dioxide gas incubator. Thereafter 10 ml of HAT medium (DMEM containing 13.6 mg/l hypoxanthine, 3.88 mg/l thymidine, 0.176 mg/l aminopterine and 10% bovine fetal serum) was substituted for the medium followed by culturing at 37°C for 2 days in a 5% carbon dioxide gas incubator. The substitution of the medium was carried out every 2 days. On Day 14, 12 colonies appeared on the dish. Each colony that appeared was transferred to a 96-cell microplate followed by culturing at 37°C in a 5% carbon dioxide gas incubator. The incubation was performed for further 2 days after the microplate had become dense. The sIL-2R in the culture supernatant in each colony was identified by the following method. As a result, sIL-2R-producing cells BTMLESS-J and BTMLESS-Q were obtained.

ATL-derived cell line MT-1 cells, on the cortex of which many IL-2Rs are expressed can be killed by treatment with anti-IL-2R monoclonal antibody (anti-Tac antibody) and a complement. Thus, a specimen containing sIL-2R is reacted with antibody, MT-1 cells are added to the reaction mixture and the resulting mixture is further treated with complement. If the sIL-2R is present in the specimen, it will bind to the antibody so that the amount of antibody to be reacted with MT-1 cells decreases. As a result, MT-1 cells will not be killed even though they are treated with the complement. Based on this principle, a trace amount (about 0.1 ng) of sIL-2R can be identified. A concrete method for identification of sIL-2R is as follows. Firstly, 10 $\mu$l of a specimen or 10 $\mu$l of a dilution of the specimen with 10% bovine fetal serum-containing RPMI 1640 medium was dissolved in the same medium in a V-shaped 96-cell microplate. The solution was thoroughly mixed with 10 $\mu$l of a 1.6 ng anti-Tac antibody solution followed by allowing to stand at 0°C for 10 minutes. A suspension of 20 $\mu$l of 5 x $10^5$/ml MT-1 cells in the above medium was added to the mixture. After mixing, the system was allowed to stand at 0°C for 30 minutes. After centrifuging at 800 rpm for 5 minutes, the supernatant was discarded and 20 $\mu$l of complement (15-fold dilution of rabbit newborn serum with 10% bovine fetal serum-containing RPMI 1640 medium) was added thereto and mixed followed by allowing to stand at 37°C for 30 minutes. Then 20 $\mu$l of a 0.5% tripane blue-containing PBS solution was added to measure the count of killed cells in 200 cells.

(4) The thus produced IL-2R in secreted form can be stored in freezing at -20°C. Even though it is concentrated by ultrafiltration (e.g., Sentricon 10), its properties are unchanged. Furthermore it can be bound to a IL-2 column. Namely, 100 $\mu$l of a specimen concentrated 10 times with Sentricon 10 is adsorbed to an IL-2-Sephadex 4B (containing 200 $\mu$ g of IL-2) column and eluted with a 0.1M sodium citrate buffer (pH 3.0) so that the sIL-2R is eluted out. The eluted fraction is immediately neutralized with 1M Tris base. The thus obtained sIL-2R inhibited the killing effect of anti-Tac antibody and complement for Mt-1 cells

Example 2

The culture supernatant of the mouse L cells containing sIL-2R protein was concentrated 10 times using Amicon hollow fibers and applied to IL-2-Sepharose 4B column chromatography to separate from other protein contaminants. Namely, the mouse L cells were cultured in 10% FCS-containing DMEM medium (100 U/ml penicillin, 100 $\mu$g/ml streptomycin and 2 g/l NaHCO$_3$) using Falcon 3027 Roller Bottle to give 15 liters of the culture supernatant. The supernatant was concentrated 10 times using Amicon hollow fibers (molecular weight, 10,000 ) to make 1500 ml, which was developed in IL-2-Sepharose 4B column of a 1 ml volume (binding 1.9 mg of IL-2 per 1 ml of gel). After development of the specimen, the column was thoroughly washed with 10 ml of PBS and then with distilled water in an amount equal to the column volume. From the IL-2R-Sepharose 4B column, the IL-2R protein was eluted with 0.1M acetic acid (pH 3.1). By this procedure, sIL-2R was purified about 15,000 times and the recovery rate was 75%. The eluted fraction of the IL-2-Sepharose 4B column was further purified by reverse phase HPLC using ODS column (Yamamura Science, 4 mm x 60 mm). The IL-2-Sepharose 4B column eluted fraction, 4 ml, was added to an ODS column which had been equlibrated with 0.1 ml of trifluoroacetic acid (pH 2.0) and the adsorbed protein was eluted by linear density gradient of acetonitrile of 0 to 80%. sIL-2R was eluted with 38 to 40% of acetonitrile. By the foregoing purification method, the sIL-2R molecule could be separated and purified finally 35,000 times from 15 liters of the culture supernatant, with the recovery rate of 50%.

[Molecular weight of IL-2R in secreted form]

Using the sIL-2R specimen after HPLC, the purity of this specimen was examined and the molecular weight was examined with SDS-PAGE. The specimen was added to 10% polyacryamide gel containing 0.1% SDS and electrophoresis was performed. As a result, a single band was given at a place corresponding to a molecular weight of 40 kd to 43 kd. As standard proteins, bovine serum albumin, ovalbumin, chymotripsinogen, and soybean trypsin inhibitor were used.

[pI]

Using the purified sIL-2R, pI was determined. The specimen was added to Mono P Column (Pharmacia Fine Chemicals, Sweden) equilibrated with 25 mM bis-Tris-iminodiacetic acid (pH 7.0) followed by eluting with polybuffer 74-iminodiacetic acid (pH 4.0). It was made clear that sIL-2R was eluted in a range showing pH of 5.2 to 4.4 and its isoelectric point was 5.2 to 4.4

[N-Terminal structure of sIL-2R]

To determine the amino acid sequence of the sIL-2R protein, purified sIL-2R was introduced in Protein Sequencer (Applied Biosystem Co.). Determination of the amino acid sequence was performed in accordance with the method described in J. Biol. Chem., 193, 265-275 (1951).

It was made clear that the sIL-2R protein had a molecule showing the following N-terminal structure:

Glu-Leu-Cys-Asp-Asp-Asp-Pro-Pro-Glu-Ile-

This structure was quite identical with the amino acid structure presumed from the base sequence of the IL-2R gene (Nature, 311, 631-635,(1984)).

[Effect]

According to this invention, IL-2R released from the cell cortex which can treat diseases induced by excessive immunity, etc. by scavenging and inactivating IL-2, can be constructed.

4. The invention is further supported by the enclosed figures.

Figure 1 is a view for explaining the progress of construction of pKCRIL2R BTMLESS.

Figure 2 is a view for explaining the progress of construction of pDRIL2R BTMLESS.

**Claims**

1. Interleukin 2 receptor in secreted form having the following properties:
   (a) molecular weight: 40 to 43 kd
   (b) pI of 5.2 to 4.4
   (c) amino acid sequence:

X- Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

```
Trp-Glu-Asn-Glu-Ala-Thr-Glu-Arg-Ile-Tyr-His-
Phe-Val-Val-Gly-Gln-Met-Val-Tyr-Tyr-Gln-Cys-
Val-Gln-Gly-Tyr-Arg-Ala-Leu-His-Arg-Gly-Pro-
Ala-Glu-Ser-Val-Cys-Lys-Met-Thr-His-Gly-Lys-
Thr-Arg-Trp-Thr-Gln-Pro-Gln-Leu-Ile-Cys-Thr-
Gly-Glu-Met-Glu-Thr-Ser-Gln-Phe-Pro-Gly-Glu-
Glu-Lys-Pro-Gln-Ala-Ser-Pro-Glu-Gly-Arg-Pro-
Glu-Ser-Glu-Thr-Ser-Cys-Leu-Val-Thr-Thr-Thr-
Asp-Phe-Gln-Ile-Gln-Thr-Glu-Met-Ala-Ala-Thr-
Met-Glu-Thr
```

wherein X represents Met or a signal peptide having Met at the N-terminus.

2. A method for the production of interleukin 2 receptor in secreted form according to claim 1 comprising culturing L cells or microorganisms belonging to the genus Escherichia, transformed with an expression vector having the following characteristics:
it carries the DNA sequence coding for the interleukin 2 receptor in secreted form having the amino acid sequence

```
X-Glu-Leu-Cys-Asp-Asp-Asp-Pro-Pro-Glu-Ile-
Pro-His-Ala-Thr-Phe-Lys-Ala-Met-Ala-Tyr-Lys-
Glu-Gly-Thr-Met-Leu-Asn-Cys-Glu-Cys-Lys-Arg-
Gly-Phe-Arg-Arg-Ile-Lys-Ser-Gly-Ser-Leu-Tyr-
Met-Leu-Cys-Thr-Gly-Asn-Ser-Ser-His-Ser-Ser-
Trp-Asp-Asn-Gln-Cys-Gln-Cys-Thr-Ser-Ser-Ala-
Thr-Arg-Asn-Thr-Thr-Lys-Gln-Val-Thr-Pro-Gln-
Pro-Glu-Glu-Gln-Lys-Glu-Arg-Lys-Thr-Thr-Glu-
Met-Gln-Ser-Pro-Met-Gln-Pro-Val-Asp-Gln-Ala-
Ser-Leu-Pro-Gly-His-Cys-Arg-Glu-Pro-Pro-Pro-
Trp-Glu-Asn-Glu-Ala-Thr-Glu-Arg-Ile-Tyr-His-
Phe-Val-Val-Gly-Gln-Met-Val-Tyr-Tyr-Gln-Cys-
Val-Gln-Gly-Tyr-Arg-Ala-Leu-His-Arg-Gly-Pro-
Ala-Glu-Ser-Val-Cys-Lys-Met-Thr-His-Gly-Lys-
Thr-Arg-Trp-Thr-Gln-Pro-Gln-Leu-Ile-Cys-Thr-
Gly-Glu-Met-Glu-Thr-Ser-Gln-Phe-Pro-Gly-Glu-
Glu-Lys-Pro-Gln-Ala-Ser-Pro-Glu-Gly-Arg-Pro-
Glu-Ser-Glu-Thr-Ser-Cys-Leu-Val-Thr-Thr-Thr-
Asp-Phe-Gln-Ile-Gln-Thr-Glu-Met-Ala-Ala-Thr-
Met-Glu-Thr
```

(wherein X represents Met or a signal peptide having Met at the N-terminal), arranged to be expressed downstream of a promotor sequence; it is capable of proliferating in the host cell; it includes a termination codon immediately after the codon for threonine at amino acid position 211 in the interleukine 2 receptor DNA.

**3.** A pharmaceutical composition, comprising the interleukin 2 receptor protein according to claim 1.

**Revendications**

**1.** Récepteur de l'interleukine 2 sous la forme sécrétée ayant les propriétés suivantes :
      (a) poids moléculaire : 40 à 43 kd
      (b) pI de 5,2 à 4,4
      (c) séquence d'amino-acides :

```
X- Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

Trp-Glu-Asn-Glu-Ala-Thr-Glu-Arg-Ile-Tyr-His-

Phe-Val-Val-Gly-Gln-Met-Val-Tyr-Tyr-Gln-Cys-

Val-Gln-Gly-Tyr-Arg-Ala-Leu-His-Arg-Gly-Pro-

Ala-Glu-Ser-Val-Cys-Lys-Met-Thr-His-Gly-Lys-

Thr-Arg-Trp-Thr-Gln-Pro-Gln-Leu-Ile-Cys-Thr-

Gly-Glu-Met-Glu-Thr-Ser-Gln-Phe-Pro-Gly-Glu-

Glu-Lys-Pro-Gln-Ala-Ser-Pro-Glu-Gly-Arg-Pro-

Glu-Ser-Glu-Thr-Ser-Cys-Leu-Val-Thr-Thr-Thr-

Asp-Phe-Gln-Ile-Gln-Thr-Glu-Met-Ala-Ala-Thr-

Met-Glu-Thr
```

dans laquelle X représente Met ou un peptide signal ayant Met à l'extrémité N-terminale.

2. Procédé pour la production du récepteur de l'interleukine 2 sous la forme sécrétée selon la revendication 1, comprenant la culture de cellules L ou de microorganismes appartenant au genre Escherichia, transformés avec un vecteur d'expression ayant les caractéristiques suivantes :

il porte la séquence d'ADN codant pour le récepteur de l'interleukine 2 sous la forme sécrétée ayant la séquence d'amino-acides suivante :

```
X-Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

Trp-Glu-Asn-Glu-Ala-Thr-Glu-Arg-Ile-Tyr-His-

Phe-Val-Val-Gly-Gln-Met-Val-Tyr-Tyr-Gln-Cys-

Val-Gln-Gly-Tyr-Arg-Ala-Leu-His-Arg-Gly-Pro-

Ala-Glu-Ser-Val-Cys-Lys-Met-Thr-His-Gly-Lys-

Thr-Arg-Trp-Thr-Gln-Pro-Gln-Leu-Ile-Cys-Thr-

Gly-Glu-Met-Glu-Thr-Ser-Gln-Phe-Pro-Gly-Glu-

Glu-Lys-Pro-Gln-Ala-Ser-Pro-Glu-Gly-Arg-Pro-


Glu-Ser-Glu-Thr-Ser-Cys-Leu-Val-Thr-Thr-Thr-

Asp-Phe-Gln-Ile-Gln-Thr-Glu-Met-Ala-Ala-Thr-

Met-Glu-Thr
```

(dans laquelle x représente Met ou un peptide signal ayant Met à l'extrémité N-terminale)
disposée pour être exprimée en aval d'une séquence promoteur ; il est capable de proliférer dans la cellule hôte ; et il comprend un codon d'arrêt immédiatement après le codon de la thréonine de la position 211 des amino-acides dans l'ADN du récepteur de l'interleukine 2.

**3.** Composition pharmaceutique comprenant la protéine récepteur de l'interleukine 2 selon la revendication 1.

**Patentansprüche**

**1.** Interleukin-2-Rezeptor in sekretierter Form mit den folgenden Eigenschaften :

EP 0 213 463 B1

(a) Molekulargewicht : 40 bis 43 kd
(b) pI von 5,2 bis 4,4
(c) Aminosäuresequenz :

X- Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

Trp-Glu-Asn-Glu-Ala-Thr-Glu-Arg-Ile-Tyr-His-

Phe-Val-Val-Gly-Gln-Met-Val-Tyr-Tyr-Gln-Cys-

Val-Gln-Gly-Tyr-Arg-Ala-Leu-His-Arg-Gly-Pro-

Ala-Glu-Ser-Val-Cys-Lys-Met-Thr-His-Gly-Lys-

Thr-Arg-Trp-Thr-Gln-Pro-Gln-Leu-Ile-Cys-Thr-

Gly-Glu-Met-Glu-Thr-Ser-Gln-Phe-Pro-Gly-Glu-

Glu-Lys-Pro-Gln-Ala-Ser-Pro-Glu-Gly-Arg-Pro-

Glu-Ser-Glu-Thr-Ser-Cys-Leu-Val-Thr-Thr-Thr-

Asp-Phe-Gln-Ile-Gln-Thr-Glu-Met-Ala-Ala-Thr-

Met-Glu-Thr

worin X Met oder ein Signalpeptid mit Met am N-Terminus darstellt.

2. Verfahren zur Herstellung von Interleukin-2-Rezeptor in sekretierter Form nach Anspruch 1, umfassend das Züchten von L-Zellen oder von zur Gattung Escherichia gehörenden Mikroorganismen, die mit einem Expressionsvektor mit den folgenden Merkmalen transformiert sind :
er trägt die für den Interleukin-2-Rezeptor in sekretierter Form mit der Aminosäuresequenz

13

```
X-Glu-Leu-Cys-Asp-Asp-Asp-Pro-Pro-Glu-Ile-

Pro-His-Ala-Thr-Phe-Lys-Ala-Met-Ala-Tyr-Lys-

Glu-Gly-Thr-Met-Leu-Asn-Cys-Glu-Cys-Lys-Arg-

Gly-Phe-Arg-Arg-Ile-Lys-Ser-Gly-Ser-Leu-Tyr-

Met-Leu-Cys-Thr-Gly-Asn-Ser-Ser-His-Ser-Ser-

Trp-Asp-Asn-Gln-Cys-Gln-Cys-Thr-Ser-Ser-Ala-

Thr-Arg-Asn-Thr-Thr-Lys-Gln-Val-Thr-Pro-Gln-

Pro-Glu-Glu-Gln-Lys-Glu-Arg-Lys-Thr-Thr-Glu-

Met-Gln-Ser-Pro-Met-Gln-Pro-Val-Asp-Gln-Ala-

Ser-Leu-Pro-Gly-His-Cys-Arg-Glu-Pro-Pro-Pro-

Trp-Glu-Asn-Glu-Ala-Thr-Glu-Arg-Ile-Tyr-His-

Phe-Val-Val-Gly-Gln-Met-Val-Tyr-Tyr-Gln-Cys-

Val-Gln-Gly-Tyr-Arg-Ala-Leu-His-Arg-Gly-Pro-

Ala-Glu-Ser-Val-Cys-Lys-Met-Thr-His-Gly-Lys-

Thr-Arg-Trp-Thr-Gln-Pro-Gln-Leu-Ile-Cys-Thr-

Gly-Glu-Met-Glu-Thr-Ser-Gln-Phe-Pro-Gly-Glu-

Glu-Lys-Pro-Gln-Ala-Ser-Pro-Glu-Gly-Arg-Pro-

Glu-Ser-Glu-Thr-Ser-Cys-Leu-Val-Thr-Thr-Thr-

Asp-Phe-Gln-Ile-Gln-Thr-Glu-Met-Ala-Ala-Thr-

Met-Glu-Thr
```

(worin X Met oder ein Signalpeptid mit Met am N-Terminus darstellt) kodierende Sequenz derart stromabwärts einer Promotorsequenz angeordnet, daß sie exprimiert wird; er ist zur Vermehrung in der Wirtszelle befähigt; er enthält ein Stopkodon unmittelbar nach dem Kodon für Threonin in Aminosäureposition 211 in der Interleukin-2-Rezeptor DNS.

3. Pharmazeutische Zusammensetzung, enthaltend das Interleukin-2-Rezeptorprotein nach Anspruch 1.

Figure 1

Figure 2